# EUROPEAN PATENT SPECIFICATION

(11) **EP 3 957 715 B1**
(45) Date of publication and mention of the grant of the patent: **28.01.2026**
(21) Application number: 20791398.9
(22) Date of filing: 16.04.2020
(51) Int. Cl.: A01N 1/147, C12N 5/073, C12N 5/075

(54) **DEVICE FOR THE CRYOPRESERVATION OF A SAMPLE OF OVA AND EMBRYOS BY MEANS OF VITRIFICATION**
VORRICHTUNG ZUR KRYOKONSERVIERUNG EINER PROBE VON EIZELLEN UND EMBRYOS MITTELS VITRIFIZIERUNG
DISPOSITIF POUR LA CRYOCONSERVATION D'UN ÉCHANTILLON D'OVULES ET D'EMBRYONS PAR VITRIFICATION

(30) Priority: 16.04.2019 ES 201930351
(43) Date of publication of application: 23.02.2022
(73) Proprietor: Universitat Politècnica de València, 46022 Valencia (ES)
(72) Inventor: MARCO JIMÉNEZ, Francisco, 46022 Valencia (ES); VICENTE ANTÓN, José Salvador, 46022 Valencia (ES); GARCÍA VALERO, Luis, 46022 Valencia (ES); GARCÍA DOMÍNGUEZ, Ximo, 46022 Valencia (ES)
(74) Representative: Pons IP
(86) International application number: PCT/ES2020/070244
(87) International publication number: WO 2020/212638

(56) References cited:
- WO-A1-2017/187543
- CN-U- 205 196 832
- US-A1- 2019 141 986
- TIERSCH NOLAN J. ET AL: "Standardized Assessment of Thin-film Vitrification for Aquatic Species : TIERSCH AND TIERSCH: TECHNICAL NOTE", NORTH AMERICAN JOURNAL OF AQUACULTURE, vol. 79, no. 4, 1 August 2017 (2017-08-01), pages 283 - 288, XP093042571, ISSN: 1522-2055, DOI: 10.1080/15222055.2017.1339153
- MARCO-JIM�NEZ FRANCISCO ET AL: "Development of Cheaper Embryo Vitrification Device Using the Minimum Volume Method", PLOS ONE, vol. 11, no. 2, 5 February 2016 (2016-02-05), pages e0148661, XP093042637, DOI: 10.1371/journal.pone.0148661
- TIERSCH NOLAN J ET AL.: "Three-Dimensional Printing of Vitrification Loop Prototypes for Aquatic Species", ZEBRAFISH, vol. 16, no. 3, 31 May 2019 (2019-05-31), United States, pages 252 - 261, XP055748823, ISSN: 1557-8542, Retrieved from the Internet <URL:https://www.lieebertpub.com/doi/full/10.1089/zeb.2017.1520> [retrieved on 20200814]
- CARDONA-COSTA J ET AL.: "Vitrification of zebrafish embryo blastomeres in microvolumes", CRYOLETTERS, vol. 28, no. 4, 30 June 2007 (2007-06-30), pages 303 - 309, XP055868866, ISSN: 0143-2044
- MAVRIDES ANDREAS ET AL.: "Cryopreservation of bovine oocytes: is cryoloop vitrification the future to preserving the female gamete?", REPRODUCTION, NUTRITION, DEVELOPMENT, vol. 42, no. 1, 1 January 2001 (2001-01-01), France, pages 73 - 80, XP055748825, ISSN: 0926-5287
- MARCO-JIMENEZ FRANCISCO ET AL.: "Development of Cheaper Embryo Vitrification Device Using the Minimum Volume Method", PLOS ONE, vol. 11, no. 2, 5 February 2016 (2016-02-05), pages e0148661, XP055302556, ISSN: 1932-6203

## Description

### FIELD OF THE INVENTION

The present invention discloses a device for the cryopreservation of ova and embryos based on the vitrification technique. During vitrification, ova and embryos are handled in the vitrification medium, the volume of which must be reduced as much as possible to ensure the success of the technique. Thus, the device disclosed by the present invention, given its geometry, generates a tangential force on the deposited sample which allows a thin film of vitrification medium to be generated for storing the ova or embryos in biobanks. Likewise, the device allows both individual and group storage, as well as being able to visualise the process under a light microscope to verify cell deposition and morphology.

### BACKGROUND OF THE INVENTION

The preservation of ova, embryos and tissues is a technique required in various fields. In reproductive medicine, since the birth of the first human being conceived through in vitro fertilisation in 1978, more than 6.5 million babies have been born using assisted reproduction technology (ART).

In recent years, and ushered in with the improved efficacy of the vitrification technique, a considerable increase in ART cycles which include vitrification has been observed, thus maximising the efficacy of the ovarian stimulation cycles, as the storage of excess ova or embryos for later use is allowed.

In human fertility treatment, both the ova and the embryos are subjected to vitrification and stored up until the most suitable time for transplantation. Among others, the use of cryopreservation allows the prevention of the so-called "suboptimal" endometrium, which occurs after applying ovarian stimulation treatments as a result of supraphysiological hormone levels, thus improving implantation and pregnancy rates. As a result, the cryopreservation of human embryos is now more important than ever for the accumulated pregnancy rate in ARTs. In livestock, and more specifically in cattle, more than 900,000 embryos are obtained annually, which for the most part are commercialised after cryopreservation. In pigs, and unlike in cattle, up until now there has not been any commercialised device that allows the commercial use of embryos. Straw supports having a different diameter and capacity, loop or sheet have been widely disclosed in the state of the art. They were developed for the purpose of obtaining ultra-rapid temperature exchange speeds that favoured the vitrification of ova and embryos. This was achieved by minimising the volume of the vitrification medium, going from the conventional use of straws of 250-125 microlitres to volumes of 1 microlitre. Thus, in 1985, Rall and Fahy achieved for the first time vitrification of mouse embryos, and vitrification then become the optimal method for the cryopreservation of ova and embryos. It was, shortly after, when Dr. Arav et al. introduced the idea of using the vitrification technique, but in a small drop that would end up being defined as "essential minimal drop size" (Arav, 1989: 1992: 2014; Arav et al., 1987: 2002). All vitrification devices today are based on this concept (Cobo et al., 2008). Currently, and as is understood, this same concept is used by other commercial devices such as, Cryotop, Cryolock, Cryotech, all guaranteed by the increase in survival results, but they do not use the technical solution of a loop. Likewise, the devices to date known by the applicant which allow operating with minimal volumes have a storage capacity limited to a small number of ova or embryos.

Thus, for example, Mavrides & Morroll mention a device of this type manufactured for the cryopreservation of bovine ova, describing a circular loop 1 mm in diameter. Other modifications allow the number of cells to be deposited on the support to increase, as occurs with the device disclosed by Cardona-Costa and Garcia-Ximenez (although the volume used is 250 microlitres), or result in the reduction of manufacture costs, at the same time providing easier handling or labelling of the sample, as described in Chinese utility model CN205196832U.

Document US2019/141986A1 (published on 16 May 2019) discloses a vessel that is provided for cryopreservation by vitrification for use in the cryopreservation by vitrification of cells or embryos. The cells or embryos are retained by a retaining part of the vessel and has recesses on the retaining part to store the cells or embryos therein. The vessel for cryopreservation by vitrification in a liquid cryogen has holes in the walls which make up the recesses which do not allow the cells or embryos to pass through the walls, but which do allow vitrification solution to pass therethrough. A kit is also provided which includes the vessel and a tube for storing the vessel. A method for cryopreservation by vitrification in a liquid cryogen is also provided. A family member of this document, WO 2017/187543 A1, was published on 2 November 2017.

In the article "Standardized Assessment of Thin-film Vitrification for Aquatic Species", published by the North American Journal of Aquaculture, 2017, vol. 79, p. 283-288, Nolan Tiersch discloses that ultra-rapid cooling under the appropriate conditions will produce vitrification, a glass-like state used to cryopreserve small sample volumes, but there are a number of major technical drawbacks impeding application of vitrification to germplasm of aquatic species. These include a lack of suitable devices, and poor reproducibility and comparability among studies due to a lack of standardization. They used 3-dimensional (3-D) printing to produce a viewing pedestal coupled with a classification system to rapidly assess frozen film quality of vitrification loops. The round loop at one end of the vitrification device had an external diameter of 6.5 mm. Classification time declined with practice from 2.1 ± 0.3 sec to 1.5 ± 0.2 sec (after 200 assessments), and assessments were consistently made in < 2.5 sec. Classifications should be reported with representative images allowing harmonization for quality control. This approach permits rapid classification and can be applied for development of methods including evaluation of vitrification solution components, concentrations of solution and target cells, and configurations and volumes of new devices. Future studies should address the custom fabrication of 3-D printed vitrification devices for use with aquatic species and other applications.

In the article "Three-Dimensional Printing of Vitrification Loop Prototypes for Aquatic Species", published in June 2019 by Zebrafish, vol. 16, number 3, p. 252-261, Nolan Tiersch discloses that vitrification is a method of cryopreservation that freezes samples rapidly, while forming an amorphous solid ("glass"), typically in small (µL) volumes. The goal of this project was to create, by three-dimensional (3D) printing, open vitrification devices based on an elliptical loop that could be efficiently used and stored. Vitrification efforts can benefit from the application of 3D printing, and to begin integration of this technology, they addressed four main variables: thermoplastic filament type, loop length, loop height, and method of loading. Our objectives were to: (1) design vitrification loops with varied dimensions; (2) print prototype loops for testing; (3) evaluate loading methods for the devices; and (4) classify vitrification responses to multiple device configurations. The basic design was an elliptical loop at the end of a handle, wherein the nominal lenghts of the different loop configurations were 15 mm, 19 mm and 23.5 mm, and the width was 2 mm. The various configurations were designed digitally using 3D CAD (Computer Aided Design) software, and prototype devices were produced with MakerBot^{®} 3D printers. The thermoplastic filaments used to produce devices were acrylonitrile butadiene styrene (ABS) and polylactic acid (PLA). Vitrification devices were characterized by the film volumes formed with different methods of loading (pipetting or submersion). Frozen films were classified to determine vitrification quality: zero (opaque, or abundant crystalline ice formation); one (translucent, or partial vitrification), or two (transparent, or substantial vitrification, glass). A published vitrification solution was used to conduct experiments. Loading by pipetting formed frozen films more reliably than by submersion, but submersion yielded fewer filling problems and was more rapid. The loop designs that yielded the highest levels of vitrification enabled rapid transfer of heat, and most often were characterized as being longer and consisting of fewer layers (height). 3D printing can assist standardization of vitrification methods and research, yet can also provide the ability to quickly design and fabricate custom devices when needed.

There is no known vitrification support with an essential minimal volume in a closed system which furthermore allows a considerable number of ova and embryos to be stored, for use in polytocous species such as pigs or rabbits. In addition, in devices existing to date, the reduction of the vitrification volume to said "essential minimal volume" requires the handler to possess considerable technical capabilities.

### DESCRIPTION OF THE INVENTION

The invention is defined in the appended claims. The present invention provides an easy-to-use device for the cryopreservation of ova and embryos by means of vitrification which, furthermore, increases the number of ova or embryos that can be stored. In other words, the surface tension generated by the vitrification solution containing the cells when they are deposited in the device object of the patent ensures a minimal volume around same, thus preventing the need to perform further operations to remove the excess vitrification solution (for example, direct suction in a micropipette, or by contact with absorbent elements). The device furthermore allows for an easy, reliable and efficient devitrification of the cells. More specifically, the device allows a minimal layer of vitrification medium around the ovum and the embryo, using the forces generated by the surface tension of the liquid, to retain and surround the cells with a minimal layer of vitrification medium, promoting even further the achievement of ultra-rapid cooling and heating speeds, which represents a considerable technical improvement over the solutions in the state of the art.

The present invention consists of a support that has a single piece and includes a slender holding portion configured for holding and labelling the sample, having an intermediate portion connected to a tip, wherein the tip has a pointed distal end and comprises a through-hole having an elliptical configuration comprising a maximum width on one of its axes of 0.6 mm. This geometry is what allows the formation of a thin film of vitrification medium, where the ova or embryos to be vitrified by direct contact with liquid nitrogen are included.

Preferably, the device comprises, furthermore, a protective sleeve intended to hermetically seal the support, thus protecting the sample against microbiological contamination during storage. The pointed distal end of the object facilitates the insertion of the support into the sleeve when operating under liquid nitrogen.

The device provides as a fundamental advantage the capacity of the support to house multiple ova and embryos with a minimal volume of vitrification solution without the need to carry out operations for removing the excess of said solution, which allows high cooling and heating speeds to be obtained. In addition, it is easy to handle and allows a simple labelling of the sample in the holding area to be performed.

The through-hole with an elliptical configuration can have a length of 8.8 mm and a width of 0.6 mm.

Preferably, the covering of the through-hole with an elliptical configuration has a maximum width of 2.5 mm and a maximum length of 20 mm.

The covering of the through-hole with an elliptical configuration can have a maximum width of 2.5 mm and a minimum length of 20 mm.

Preferably, the outer axes of the covering of the through-hole with an elliptical configuration have measurements of 1.8 mm and 10 mm. The support longitudinally measures between 60 and 140 mm, wherein the intermediate portion measures about 30-50% of the length of the support, and the slender holding portion measures between about 30-40% of the length of the support.

Preferably, the slender holding portion is rectangular with a length to width ratio of 10. The width can be 3 mm and the length 30 mm. The intermediate portion can be frustoconical.

Preferably, the rectangular slender holding portion has a length to width ratio of 8 to 12.

Preferably, the through-hole with an elliptical configuration at the tip has a width of 0.6 mm and a length of 10 mm, with a covering on the outside thereof with a width of 1.8 mm, the intermediate portion frustoconical measures 65 mm and the holding portion 30 mm.

In addition, the support comprises a protective sleeve intended to hermetically close the device to prevent the infection/microbial and viral contamination thereof during storage. In addition, the arrow-shaped termination of the support facilitates the insertion of the protective sleeve under the liquid nitrogen.

The device is easy to use and because of its geometry, it allows the ova and embryos to be surrounded by the minimal volume (essential minimal volume), which contributes to generating high speeds to achieve the vitreous state. This furthermore entails great ease-of-use by the handler since, once the sample is deposited on the support, no additional steps are required to reduce the volume of cryoprotective solution. Likewise, the device allows both individual storage and the storage of a large number of ova or embryos. The holding portion of the support allows labelling with the relevant information being easy to visualise. In addition, the geometry of the holding portion ensures a hermetic closure with the protective sleeve. In this manner, direct contact of the biological sample with the biobank liquid nitrogen during storage is prevented, thus preventing potential microbiological/viral contamination thereof.

The device can be used, furthermore, for the cryopreservation of spermatozoids and somatic cells.

### EXAMPLE

The support has been validated with rabbit embryos, demonstrating its functionality both in the in vitro development of said embryos and in the number of animals born. To that end, 15 female donors were used, and after receiving superovulation treatment (3 µg of Corifollitropin alfa), there were artificially inseminated (AI). After 3 days, the embryos were retrieved and cryopreserved by means of vitrification according to the protocol developed by Vicente et al. (1999), using both the device object of the patent and 2 commercial devices: Cryotop (Kitazato Co., Fuji, Japan) as the most widely used system in humans, and the 0.125 mL ministraw (French ministraw, IMV, L'Aigle, France) as the conventional system used in rabbits. Briefly, vitrification was performed in two steps. In the first step, the embryos are deposited in a solution containing 12.5% (v/v) of dimethylsulphoxide (DMSO) and 12.5% (v/v) of ethylene glycol (EG) as cryoprotective agents for 2 minutes. In the second step, the embryos are deposited in a solution containing a concentration of cryoprotective agents of 20% of DMSO and 20% of EG, the embryos being retrieved after 45 seconds in order to be deposited in the vitrification devices, and to then be submerged directly in liquid nitrogen, ensuring that incubation with the vitrification solution does not exceed 1 minute. After storage in liquid nitrogen tanks, the embryos were devitrified, evaluating the effect of the device in both in vitro conditions (embryonic development) and in vivo conditions (born individuals). The vitrification medium was removed by washing the embryos in a Dulbecco's phosphate-buffered saline (DPBS) with sucrose (0.33 M) for 5 minutes, followed by a bath in a single DPBS solution for another 5 minutes.

Next, the effects of the device on in vitro development for experimentation are described in detail. The number of embryos loaded into each of the devices was 25 for the device object of the patent, 6 for each Cryotop^{®} and 25 in each ministraw. Thus, a total of 216 vitrified embryos (65 with the device object of the patent, 68 with Cryotop^{®} and 61 with ministraw), and 22 non-vitrified embryos (control) were cultured through 7 experimental sessions for 48 h in TCM199 culture medium containing 10% foetal bovine serum (vv) (FBS) and 1% (vol / vol) antibiotics (penicillin G sodium 300 000 IU / L, penicillin G procaine 700 000 IU / L and dihydrostreptomycin sulphate 1250 mg / L; Divasa Farmavic, Barcelona, Spain) at 38.5°C and 5% CO₂ in a wet atmosphere. The development capacity up to the blastocyst hatching stage was recorded for analysis.

Next, the effects of the device on the percentage of animals born during experimentation are described in detail. A total of 288 vitrified embryos (102 with the device object of the patent, 100 Cryotop^{®} and 86 mini-straw) and 96 fresh embryos were transferred to 25 nulliparous females. The embryos were transferred via the oviduct by means of laparoscopy (Besenfelder and Brem, 1993). During laparoscopy, the recipients were anaesthetised with an intramuscular injection of xylazine (5 mg/kg), followed after 5 minutes by an injection intravenous of ketamine hydrochloride (35 mg/kg). During laparoscopy, a dose of morphine hydrochloride was administered by intramuscular route (3 mg/kg). After surgery ended, the animals were treated with antibiotics (4 mg/kg of gentamicin every 24 h) and analgesics (0.03 mg/kg of buprenorphine hydrochloride every 12 h and 0.2 mg/kg of meloxicam every 24 h) for 3 days.

The rates of embryo development up to the blastocyst hatching stage after 48 h of culture in vitro were similar between the experimental groups (89.2%, 88.2%, 90.9% and 93.4%, for the device object of the patent, Cryotop^{®}, ministraw and control group, respectively, P <0.05).

As for the offspring rate, this was similar between the groups of vitrification devices, but as was expected, significantly lower than the control group (61±4.8%, 65±4.8%, 52±4.5% vs. 74±5.2%, for the device object of the patent, Cryotop^{®}, ministraw vs. control embryos, respectively, P <0.05).

### DESCRIPTION OF THE DRAWINGS

To complement the description that is being made and for the purpose of helping to better understand the features of the invention, according to a preferred practical exemplary embodiment thereof, a set of drawings is attached as an integral part of said description, wherein the following has been depicted with an illustrative and non-limiting character:
Figure 1 shows a perspective view of the support of the device for the cryopreservation of a sample of ova or embryos by means of vitrification.
Figure 2 shows a perspective view of the support and of the protective sleeve of the device for the cryopreservation of a sample of ova or embryos by means of vitrification.
Figure 3 shows a detailed view of the deposition area of the cells at the tip of the support in the through-hole with an elliptical configuration.

### PREFERRED EMBODIMENT OF THE INVENTION

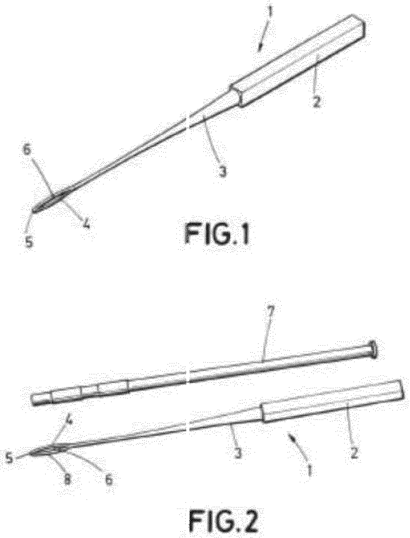

Figure 1 shows a perspective view of the support (2) of the device for the cryopreservation of a sample of ova or embryos by means of vitrification according to a preferred embodiment where it is clearly observed that the support is a single piece made of injected or 3D printed plastic, and comprising a slender holding portion (2) configured for holding and labelling the sample which is joined to an intermediate portion (3) connected to a tip (4).

Furthermore, it is shown that the tip (4) has a pointed distal end (5) and comprises a through-hole (6) having an elliptical configuration comprising a maximum width on one of its axes of 0.6 mm intended to form a thin film of vitrification medium on which the ova or embryos to be vitrified by direct contact with liquid nitrogen are deposited.

According to the preferred embodiment described in Figure 2, the through-hole (6) with an elliptical configuration (4) has a length of 10 mm, with an outer covering (8) with a width of 1.8 mm and also defining an elliptical configuration. The intermediate portion is frustoconical and substantially measures 40 mm. The slender holding portion (2) is rectangular and has a length of 30 mm and a width of 3 mm. The total length of the support is 70 mm.

Figure 2 shows a perspective view of the support (1) and of the protective sleeve (7) of the device where it is observed that the protective sleeve (7) is intended to hermetically house the support (1), and the pointed distal end (5) facilitates the insertion of the support into the sleeve (7) when operating under liquid nitrogen, and said sleeve (7) further protecting the sample from potential infection/microbial and viral contamination during storage in the biobank.

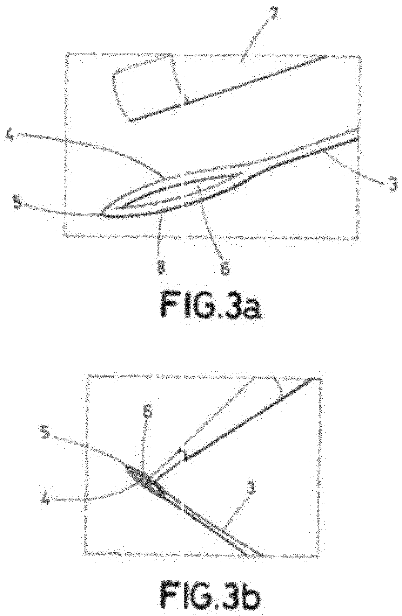

Figure 3 shows a detailed view of the deposition area of the ova or embryos at the tip of the support (1) in the through-hole (6) with an elliptical configuration, using the surface tension generated by the vitrification solution containing the ova or embryos when deposited in the device, ensuring a minimal volume around same and preventing the need to perform further operations to remove the excess vitrification solution (for example, direct suction in a micropipette, or by contact with absorbent elements). In addition, the device allows for an easy, reliable and efficient thawing of the ova and embryos.

The device allows a minimal layer of vitrification medium around the ovum and the embryo, using the forces generated by the surface tension of the liquid, to retain and surround the cells with a minimal layer of vitrification medium, promoting even further the achievement of ultra-rapid cooling and heating speeds, which represents a considerable technical improvement over the solutions in the state of the art.

The device is easy to use and because of its geometry, it allows the ova or embryos to be surrounded by the minimal volume (essential minimal volume), which contributes to generating high speeds to achieve the vitreous state. This furthermore entails great ease-of-use by the handler since, once the sample is deposited on the support, no further steps are required to reduce the volume of cryoprotective solution. Likewise, the device allows both individual storage and the storage of a large number of ova or embryos. In Figure 2, the holding portion (2) of the support (1) allows labelling with the relevant information being easy to visualise. In addition, the geometry of the holding portion (2) ensures a hermetic closure with the protective sleeve (7). In this manner, direct contact of the biological sample with the storage bank nitrogen is prevented, thus preventing potential infection/microbial and viral contamination during storage in the biobank. The pointed finish of the device at the tip (5) facilitates the insertion thereof into the sleeve when operating under immersion of the liquid nitrogen used for vitrification.

## Claims

1. A device for the cryopreservation of a sample of ova or embryos by means of vitrification, which comprises a support (1) that has a single piece and includes a slender holding portion (2) configured for holding and labelling the sample and which is joined to an intermediate portion (3) connected to a tip (4), wherein said tip (4) has a pointed distal end (5) and comprises a through-hole (6) having an elliptical configuration comprising a maximum width on one of its axes of 0.6 mm intended to form a thin film of vitrification medium on which the ova or embryos to be vitrified by direct contact with liquid nitrogen are deposited.

2. The device for the cryopreservation of ova or embryos according to claim 1, **characterised in that** it comprises a protective sleeve (7) intended to hermetically house the support (1), where the pointed distal end (5) facilitates the insertion of the support into the sleeve (7) when operating under liquid nitrogen and said sleeve (7) protecting from potential infection/microbial and viral contamination of the sample during storage.

3. The device for the cryopreservation of ova or embryos according to claim 1, **characterised in that** the tip (4) has an outer covering (8) of the through-hole (6) that has an elliptical configuration, with a width of 1.8 mm and a length of 10 mm.

4. The device for the cryopreservation of ova or embryos according to claim 1, **characterised in that** the support (1) is made of a plastic material suitable for injection and/or 3D printing.

5. The device for the cryopreservation of ova or embryos according to claim 1, **characterised in that** the support (1) longitudinally measures between 60-140 mm.

6. The device for the cryopreservation of ova or embryos according to claim 5, **characterised in that** the intermediate portion (3) is frustoconical and longitudinally measures 30-50% of the total length of the support.

7. The device for the cryopreservation of ova or embryos according to claim 5, **characterised in that** the slender holding portion (2) substantially measures 30-40% of the length of the support.

8. The device for the cryopreservation of ova or embryos according to claim 1, **characterised in that** the slender holding portion (2) is rectangular with a length to width ratio of 8 to 12.

9. The device for the cryopreservation of ova or embryos according to claim 1, **characterised in that** the slender holding portion (2) is rectangular and comprises a width of 3 mm and a length of 30 mm.

## Patentansprüche

1. Vorrichtung zur Kryokonservierung einer Probe von Eizellen oder Embryos mittels Vitrifizierung, die einen Träger (1) umfasst, der einstückig ist und einen schlanken Halteabschnitt (2) beinhaltet, der dazu konfiguriert ist, die Probe zu halten und zu etikettieren, und der an einen Zwischenabschnitt (3) angeschlossen ist, der mit einer Spitze (4) verbunden ist, wobei die Spitze (4) ein spitzes distales Ende (5) aufweist und ein Durchgangsloch (6) mit einer elliptischen Konfiguration umfasst, das eine maximale Breite auf einer seiner Achsen von 0,6 mm zur Bildung eines dünnen Films aus Vitrifizierungsmedium umfasst, auf dem die Eizellen oder Embryos, die durch direkten Kontakt mit flüssigem Stickstoff zu vitrifizieren sind, aufgetragen werden.

2. Vorrichtung zur Kryokonservierung von Eizellen oder Embryos nach Anspruch 1, **dadurch gekennzeichnet, dass** sie eine Schutzhülse (7) umfasst, die dazu bestimmt ist, den Träger (1) hermetisch aufzunehmen, wobei das spitze distale Ende (5) das Einfügen des Trägers in die Hülse (7) erleichtert, wenn unter flüssigem Stickstoff gearbeitet wird, und wobei die Hülse (7) vor einer potenziellen Infektion/mikrobiellen und viralen Verunreinigung der Probe während der Lagerung schützt.

3. Vorrichtung zur Kryokonservierung von Eizellen oder Embryos nach Anspruch 1, **dadurch gekennzeichnet, dass** die Spitze (4) eine äußere Abdeckung (8) des Durchgangslochs (6) aufweist, die eine elliptische Konfiguration mit einer Breite von 1,8 mm und einer Länge von 10 mm aufweist.

4. Vorrichtung zur Kryokonservierung von Eizellen oder Embryos nach Anspruch 1, **dadurch gekennzeichnet, dass** der Träger (1) aus einem Kunststoffmaterial hergestellt ist, das für Injektion und/oder 3D-Druck geeignet ist.

5. Vorrichtung zur Kryokonservierung von Eizellen oder Embryos nach Anspruch 1, **dadurch gekennzeichnet, dass** der Träger (1) in Längsrichtung zwischen 60-140 mm misst.

6. Vorrichtung zur Kryokonservierung von Eizellen oder Embryos nach Anspruch 5, **dadurch gekennzeichnet, dass** der Zwischenabschnitt (3) kegelstumpfförmig ist und in Längsrichtung 30-50 % der Gesamtlänge des Trägers misst.

7. Vorrichtung zur Kryokonservierung von Eizellen oder Embryos nach Anspruch 5, **dadurch gekennzeichnet, dass** der schlanke Halteabschnitt (2) im Wesentlichen 30-40 % der Länge des Trägers misst.

8. Vorrichtung zur Kryokonservierung von Eizellen oder Embryos nach Anspruch 1, **dadurch gekennzeichnet, dass** der schlanke Halteabschnitt (2) rechteckig ist mit einem Verhältnis von Länge zu Breite von 8 bis 12.

9. Vorrichtung zur Kryokonservierung von Eizellen oder Embryos nach Anspruch 1, **dadurch gekennzeichnet, dass** der schlanke Halteabschnitt (2) rechteckig ist und eine Breite von 3 mm und eine Länge von 30 mm umfasst.

## Revendications

1. Dispositif de cryoconservation d'un échantillon d'ovules ou d'embryons au moyen d'une vitrification, qui comprend un support (1) qui est d'un seul tenant et comporte une partie de préhension élancée (2) conçue pour la préhension et l'étiquetage de l'échantillon et qui est reliée à une partie intermédiaire (3) connectée à une pointe (4), dans lequel ladite pointe (4) a une extrémité distale pointue (5) et comprend un trou traversant (6) ayant une configuration elliptique comprenant une largeur maximale sur l'un de ses axes de 0,6 mm destiné à former une fine pellicule de milieu de vitrification sur laquelle sont déposés les ovules ou les embryons à vitrifier par contact direct avec de l'azote liquide.

2. Dispositif de cryoconservation d'ovules ou d'embryons selon la revendication 1, **caractérisé en ce qu'**il comprend un manchon de protection (7) destiné à loger hermétiquement le support (1), l'extrémité distale pointue (5) facilitant l'insertion du support dans le manchon (7) lorsqu'il est utilisé sous azote liquide et ledit manchon (7) protégeant l'échantillon d'une éventuelle infection/contamination microbienne et virale pendant le stockage.

3. Dispositif de cryoconservation d'ovules ou d'embryons selon la revendication 1, **caractérisé en ce que** la pointe (4) a un revêtement externe (8) du trou traversant (6) qui a une configuration elliptique, avec une largeur de 1,8 mm et une longueur de 10 mm.

4. Dispositif de cryoconservation d'ovules ou d'embryons selon la revendication 1, **caractérisé en ce que** le support (1) est en matière plastique adaptée à l'injection et/ou à l'impression 3D.

5. Dispositif de cryoconservation d'ovules ou d'embryons selon la revendication 1, **caractérisé en ce que** le support (1) mesure longitudinalement entre 60 et 140 mm.

6. Dispositif de cryoconservation d'ovules ou d'embryons selon la revendication 5, **caractérisé en ce que** la partie intermédiaire (3) est tronconique et mesure longitudinalement 30 à 50 % de la longueur totale du support.

7. Dispositif de cryoconservation d'ovules ou d'embryons selon la revendication 5, **caractérisé en ce que** la partie de préhension élancée (2) mesure sensiblement 30 à 40 % de la longueur du support.

8. Dispositif de cryoconservation d'ovules ou d'embryons selon la revendication 1, **caractérisé en ce que** la partie de préhension élancée (2) est rectangulaire avec un rapport longueur sur largeur de 8 à 12.

9. Dispositif de cryoconservation d'ovules ou d'embryons selon la revendication 1, **caractérisé en ce que** la partie de préhension élancée (2) est rectangulaire et comprend une largeur de 3 mm et une longueur de 30 mm.
